# EUROPEAN PATENT SPECIFICATION

(11) **EP 4 724 114 B1**
(45) Date of publication and mention of the grant of the patent: **11.11.2026**
(21) Application number: 25759761.7
(22) Date of filing: 18.08.2025
(51) Int. Cl.: A61L 24/06

(54) **CYANOACRYLATE ADHESIVES FOR WOUND CLOSURE**
CYANOACRYLAT-KLEBSTOFFE ZUM WUNDVERSCHLUSS
ADHÉSIFS CYANOACRYLATES POUR LA FERMETURE DES PLAIES

(30) Priority: 20.08.2024 US 202463685116 P; 24.10.2024 GB 202415677
(43) Date of publication of application: 15.04.2026
(73) Proprietor: Chemence, Inc., Alpharetta, Georgia 30005 (US)
(72) Inventor: TANG, Houliang, Cumming, Georgia 30041 (US); ALI, Faisal, Naperville, Illinois 60564 (US); KIM, David, Alpharetta, Georgia 30005 (US); SEO, Kwang, Johns Creek, Georgia 30097 (US)
(74) Representative: HGF
(86) International application number: PCT/GB2025/051820
(87) International publication number: WO 2026/041855

(56) References cited:
- JP-B2- 6 342 155
- US-A1- 2008 003 196
- US-A1- 2010 030 258
- US-B2- 8 475 825

## Description

### Technical field of invention:

The present invention relates to cyanoacrylate adhesive kits and the constituent components thereof. The cyanoacrylate adhesive kits of the present invention may be useful in the field of wound closure adhesives. In particular, the present invention relates to highly reactive initiators that allow for rapid and/or controllable cyanoacrylate curing times. In particular, the present invention relates to a kit with a single initiator or dual initiators to allow cyanoacrylate cure within a desire timeframe. In particular, the present invention relates to cyanoacrylate adhesive kits that have a lower level of toxicity as compared with conventional cyanoacrylate adhesive kits. In particular, the present invention relates to cyanoacrylate adhesive kits that reduce the risk of allergic contact dermatitis in patients, as compared with cyanoacrylate adhesive kits.

### Background:

Cyanoacrylates have been widely used in the healthcare industry as wound closure adhesives. Cyanoacrylates are reactive monomers that undergo polymerisation through initiation on contact with anions (anionic polymerisation) as well as non-dissociated bases (zwitterionic polymerisation). Initiation can occur as a result of the presence of adventitious moisture present on the surface of the skin, however relying on this mechanism alone can lead to slow curing time. The curing time is of great importance for health professionals, with preferred curing times typically ranging from 30-120 s. In order for cyanoacrylate adhesives to be viable wound closure adhesives, additional initiator components are often required.

Known attempts to improve the curing time, use quaternary ammonium salts as the initiator and optionally an accelerator or a curing rate modifier to control the curing speed, such as commercially available the DermaBond Advanced^{™} topical skin adhesive manufactured by Ethicon. The quaternary ammonium chloride and bromide salts as polymerization initiators are preferred. Examples include domiphen bromide, butyrylcholine chloride, benzalkonium bromide, benzalkonium chloride, acetyl choline chloride, etc. When the benzalkonium halide is used, it may be benzalkonium halide in its unpurified state, which comprises a mixture of varying chain length compounds, or it may be any suitable purified compound including those having a chain length of form about 12 to about 18 carbon atoms. As a medical device for wound closure application, the initiator and accelerator are typically impregnated into/onto a porous pellet inside the applicator. In use, the adhesive is pushed to pass through the pellet and contacted with the initiator., Then, the cyanoacrylate-based adhesive undergoes rapid polymerization and form a polymer film on the site within minutes. After the application, the quaternary ammonia salt and accelerators or curing rate modifiers would stay in the cured adhesive film 7-14 days, which however, may lead to an increasing number of adverse skin reactions, such as allergy contact dermatitis (ACD). These reactions are likely to be a result of the toxic nature of both the quaternary ammonium salts and the accelerators.

One such example of an adhesive composition including a cyanoacrylate monomer and an initiator component is US8475825B2. US8475825B2 discloses various classes of cyanoacrylate initiators including, but not limited to, quaternary ammonium salts (QAS); tertiary amines; and inner salts; and amines, imines and amides (such as imidazole). Suitable quaternary ammonium salts include tetraalkylammonium halides, ether amine quaternaries, quaternary ammonium sulfate salts, quaternary ammonium bisulfate salts and benzalkonium chloride. Whilst further preferred among the QAS's are sulfate and bisulfate salts. The authors of this document report the production of a biocompatible adhesive composition for use on living tissue. The curable composition described in this document includes: a non-porous substrate consisting of a plurality of individual particulates; an initiator component; and a prepolymer composition comprising at least one liquid cyanoacrylate monomer or mixture of monomers and/or cyanoacrylate oligomers. The initiator is deposited on the surface of the non-porous substrate.

US9353299B2 discloses a 2-cyanoacrylate based adhesive composition, containing a 2-cyanoacrylic acid ester as the principal component, for use on metals and thermoplastic elastomers which are low in polarity. The 2-cyanoacrylate acid ester is blended with an onium salt (the initiator) having a specific structure (C⁺A⁻). The cation of the onium salt is selected from a quaternary ammonium cation, an imidazolium cation, a pyridinium cation or a tertiary sulfonium cation (as well as a pyrrolidinium cation or a phosphonium cation). The anion of the onium salt is selected from a hydrogen sulfate, bisulfate or a sulfonate anion. The authors propose that the onium salt releases an anion when it reacts with salts present on the surface of the substrate which act as the polymerisation initiator. Since these salts are only present on the surface of the metal and thermoplastic substrate, the cyanoacrylate component and the onium salt can be stored together as a mixture, without polymerisation occurring. Initiators with hydrogen sulfate, bisulfate or sulfonate anions are unsuitable for use in wound closure adhesives since they would be corrosive to the skin (particularly hydrogen sulphates).

US5262200A discloses an apparatus for the application of a controlled amount of cyanoacrylate adhesive solution to a substrate, for use in the screen printing industry. It includes an adsorbent, wicking material that is permeated with the catalyst (the initiator) solution, allowing for controlled transfer of the catalyst solution from the applicator to a portion of the substrate. Suitable catalysts are disclosed in US5262200A are aromatic amines (including N,N-dimethylparatoluidine and N,N-diisopropylparatoluidine) that accelerate the anionic polymerisation process. The catalyst solution is deposited onto the substrate prior to the cyanoacrylate adhesive. The primary use of this invention is in the screen printing industry to bond meshed screen fabric to the peripheral surface of a screen frame structure. The authors report that the apparatus allows for the convenient use of low volatility solvents to dissolve the catalyst (rather than more toxic VOCs previously used in the art), which must be applied in small amounts. These initiators are unsuitable for use in wound closure adhesives as they are highly toxic compounds, and in the apparatus disclosed in US5262200A, the catalyst solution further comprises a toxic organic solvent. Additionally, in US5262200A the apparatus/method does not allow for the simultaneous application of the cyanoacrylate adhesive and the initiator components, which is inconvenient to the healthcare industry.

US6455064B1 discloses the use of a "medicament" which acts as both an initiator and/or accelerator of polymerisation of a monomeric adhesive composition. The adhesive compositions in this disclosure are directed for use in medical applications, including as wound closure adhesives. Medicaments are preferably a number of pharmaceutically active materials (such as antibiotics and antimicrobials), but also disclosed are quaternary ammonium halide salts, such as alkylbenzyldimethylammonium chloride and benzethonium chloride. In this document, the medicament is applied first to a tissue, followed by a polymerizable monomer composition.

Szanka, Istvan, Amalia Szanka, and Joseph P. Kennedy. "Rubbery wound closure adhesives. II. initiators for and initiation of 2-octyl cyanoacrylate polymerization." Journal of Polymer Science Part A: Polymer Chemistry 53.14 (2015): 1652-1659, describe the suitability and reactivity of five tertiary amines as initiators for neat 2-octyl cyanoacrylate polymerisation as wound closure adhesives. The authors of this document reported that N,N-dimethyl-p-toluidine is the least reactive of the five, whilst pyridine, triethylamine, azabicyclo[2.2.2]octane and diazabicyclo[2.2.2]octane are all reactive initiators. Once again, however, all five of the initiators investigated in this study are toxic and should not be used for medical applications (particularly N,N-dimethyl-p-toluidine, triethylamine and azabicyclo[2.2.2]octane).

Pepper, David Charles, and Bernard Ryan. "Initiation processes in polymerizations of alkyl cyanoacrylates by tertiary amines: inhibition by strong acids." Die Makromolekulare Chemie: Macromolecular Chemistry and Physics 184.2 (1983): 383-394 and Pepper, David Charles, and Bernard Ryan. "Kinetics of polymerization of alkyl cyanoacrylates by tertiary amines and phosphines." Die Makromolekulare Chemie: Macromolecular Chemistry and Physics 184.2 (1983): 395-410 have investigated the reactivity of several tertiary amines, including pyridine (and substituted derivatives), benzyldiethylamine and triphenylphosphine as initiators in alkyl cyanoacrylate polymerisation reactions.

Eromosele, Ighodalo Clement, and David Charles Pepper. "Anionic polymerization of butyl cyanoacrylate by tetrabutylammonium salts, 1. Initiation processes." Die Makromolekulare Chemie: Macromolecular Chemistry and Physics 190.12 (1989): 3085-3094 discloses the use of tetrabutylammonium salts (i.e. a quaternary ammonium salt) of acetate, cyanoacetate, chloroacetates and bromide.

US 2008/003196 discloses an adhesive composition including one or more polymerizable cyanoacrylate monomers and boron trifluoride as a stabilizer or complexing agent. The adhesive composition may also include or be used with a decomplexing agent, particularly one or more quaternary ammonium fluoride salts or one or more quaternary ammonium ether salts.

US 2010/030258 discloses an adhesive composition including one or more polymerizable cyanoacrylate monomers, a polymerization initiator and a polymerization accelerator for the one or more polymerizable cyanoacrylate monomers. The initiator may be a quaternary ammonium salt, and the accelerator may be a trihydroxy tertiary amine.

It would be advantageous to identify additional reactive initiators that allow for improved control over the curing rate of cyanoacrylate wound closure adhesives, particularly 2-octyl cyanoacrylate based wound closure adhesives. It would also be advantageous to identify initiators that are non-toxic, as this would reduce the risk of adverse health effects in patients to which the adhesive is applied.

None of the prior art that is known to the applicant provides for cyanoacrylate adhesive kit that is both (i) highly reactive, providing fast and controllable curing times; (ii) non-toxic and suitable for use as a wound closure adhesive. The present invention addresses this need.

### Summary of invention:

According to a first aspect there is provided a wound closure adhesive kit comprising:
(A) an adhesive component comprising a cyanoacrylate; and
(B) an initiator component comprising a tertiary amine salt of formula (II): 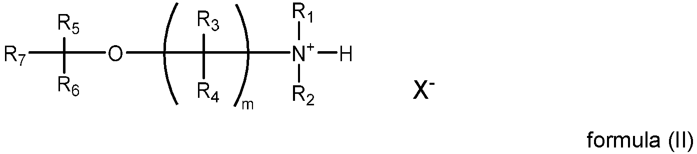 wherein:
   R₁ and R₂ are each independently selected from the group consisting of: C₁₋₂₀-alkyl, aryl and heteroaryl; or
   R₁ and R₂, together with the nitrogen atom to which they are attached, form a heterocycloalkyl ring having from 5 to 8 atoms;
   R₃ and R₄ are both H;
   R₅ and R₆ are each independently selected from the group consisting of: aryl and heteroaryl;
   m is 1 - 6;
      wherein any of the alkyl, heterocycloalkyl, aryl or heteroaryl groups of R₁ to R₆, can be optionally substituted with from 1 to 3 independently selected groups selected from: halo, C₁₋₄-alkyl and -(CH₂)_{q}-phenyl; wherein q is 0 - 2;
   R₇ is selected from the group consisting of: H and C₁₋₂₀-alkyl; and
   X⁻ is a counterion.

It may be that the kit is a topical adhesive kit.

In some embodiments the kit comprises one tertiary amine salt described herein. It may be that the kit comprises a mixture of more than one tertiary amine salt described herein. For example, the kit may comprise a mixture of two tertiary amine salts. For example, the kit may comprise a mixture of three tertiary amine salts.

It may be that the kit further comprises an applicator in which the at least one tertiary amine salt is contained.

In an embodiment, R₁ and R₂ are each independently selected from the group consisting of: C₁₋₁₅-alkyl, 6-10 membered aryl and 5-10 membered heteroaryl.

In an embodiment, R₁ and R₂ are each independently selected from the group consisting of: C₁₋₁₂-alkyl, 6-10 membered aryl and 5-10 membered heteroaryl.

In an embodiment, R₁ and R₂ are each independently selected from the group consisting of: C₁₋₁₀-alkyl, 6-10 membered aryl and 5-10 membered heteroaryl.

In an embodiment, R₁ and R₂ are each independently selected from the group consisting of: C₁₋₈-alkyl, 6-10 membered aryl and 5-10 membered heteroaryl.

In an embodiment, R₁ and R₂ are each independently selected from the group consisting of: C₁₋₆-alkyl, 6-10 membered aryl and 5-10 membered heteroaryl.

In an embodiment, R₁ and R₂ are each independently selected from the group consisting of: C₁₋₄-alkyl, 6-10 membered aryl and 5-10 membered heteroaryl.

In an embodiment, R₁ and R₂ are each independently selected from the group consisting of: C₁₋₃-alkyl, 6-10 membered aryl and 5-10 membered heteroaryl.

In an embodiment, R₁ and R₂ are each independently selected from the group consisting of: C₁₋₂-alkyl, 6-10 membered aryl and 5-10 membered heteroaryl.

In an embodiment, R₁ and R₂ are each independently selected from the group consisting of: methyl, 6-10 membered aryl and 5-10 membered heteroaryl.

In an embodiment, R₁ and R₂ are each independently selected from the group consisting of: C₁₋₁₅-alkyl, 6-membered aryl and 5-6 membered heteroaryl.

In an embodiment, R₁ and R₂ are each independently selected from the group consisting of: C₁₋₁₂-alkyl, 6-membered aryl and 5-6 membered heteroaryl.

In an embodiment, R₁ and R₂ are each independently selected from the group consisting of: C₁₋₁₀-alkyl, 6-membered aryl and 5-6 membered heteroaryl.

In an embodiment, R₁ and R₂ are each independently selected from the group consisting of: C₁₋₈-alkyl, 6-membered aryl and 5-6 membered heteroaryl.

In an embodiment, R₁ and R₂ are each independently selected from the group consisting of: C₁₋₆-alkyl, 6-membered aryl and 5-6 membered heteroaryl.

In an embodiment, R₁ and R₂ are each independently selected from the group consisting of: C₁₋₄-alkyl, 6-membered aryl and 5-6 membered heteroaryl.

In an embodiment, R₁ and R₂ are each independently selected from the group consisting of: C₁₋₃-alkyl, 6-membered aryl and 5-6 membered heteroaryl.

In an embodiment, R₁ and R₂ are each independently selected from the group consisting of: C₁₋₂-alkyl, 6-membered aryl and 5-6 membered heteroaryl.

In an embodiment, R₁ and R₂ are each independently selected from the group consisting of: methyl, 6-membered aryl and 5-6 membered heteroaryl.

In an embodiment, R₁ and R₂ are each independently selected from the group consisting of: C₁₋₁₅-alkyl, phenyl and pyridinyl.

In an embodiment, R₁ and R₂ are each independently selected from the group consisting of: C₁₋₁₂-alkyl, phenyl and pyridinyl.

In an embodiment, R₁ and R₂ are each independently selected from the group consisting of: C₁₋₁₀-alkyl, phenyl and pyridinyl.

In an embodiment, R₁ and R₂ are each independently selected from the group consisting of: C₁₋₈-alkyl, phenyl and pyridinyl.

In an embodiment, R₁ and R₂ are each independently selected from the group consisting of: C₁₋₆-alkyl, phenyl and pyridinyl.

In an embodiment, R₁ and R₂ are each independently selected from the group consisting of: C₁₋₄-alkyl, phenyl and pyridinyl.

In an embodiment, R₁ and R₂ are each independently selected from the group consisting of: C₁₋₃-alkyl, phenyl and pyridinyl.

In an embodiment, R₁ and R₂ are each independently selected from the group consisting of: C₁₋₂-alkyl, phenyl and pyridinyl.

In an embodiment, R₁ and R₂ are each independently selected from the group consisting of: methyl, phenyl and pyridinyl.

In an embodiment, R₁ and R₂ are each independently selected from the group consisting of: C₁₋₁₅-alkyl and 6-10 membered aryl.

In an embodiment, R₁ and R₂ are each independently selected from the group consisting of: C₁₋₁₂-alkyl and 6-10 membered aryl.

In an embodiment, R₁ and R₂ are each independently selected from the group consisting of: C₁₋₁₀-alkyl and 6-10 membered aryl.

In an embodiment, R₁ and R₂ are each independently selected from the group consisting of: C₁₋₈-alkyl and 6-10 membered aryl.

In an embodiment, R₁ and R₂ are each independently selected from the group consisting of: C₁₋₆-alkyl and 6-10 membered aryl.

In an embodiment, R₁ and R₂ are each independently selected from the group consisting of: C₁₋₄-alkyl and 6-10 membered aryl.

In an embodiment, R₁ and R₂ are each independently selected from the group consisting of: C₁₋₃-alkyl and 6-10 membered aryl.

In an embodiment, R₁ and R₂ are each independently selected from the group consisting of: C₁₋₂-alkyl and 6-10 membered aryl.

In an embodiment, R₁ and R₂ are each independently selected from the group consisting of: methyl and 6-10 membered aryl.

In an embodiment, R₁ and R₂ are each independently selected from the group consisting of: C₁₋₁₅-alkyl and 6-membered aryl.

In an embodiment, R₁ and R₂ are each independently selected from the group consisting of: C₁₋₁₂-alkyl and 6-membered aryl.

In an embodiment, R₁ and R₂ are each independently selected from the group consisting of: C₁₋₁₀-alkyl and 6-membered aryl.

In an embodiment, R₁ and R₂ are each independently selected from the group consisting of: C₁₋₈-alkyl and 6-membered aryl.

In an embodiment, R₁ and R₂ are each independently selected from the group consisting of: C₁₋₆-alkyl and 6-membered aryl.

In an embodiment, R₁ and R₂ are each independently selected from the group consisting of: C₁₋₄-alkyl and 6-membered aryl.

In an embodiment, R₁ and R₂ are each independently selected from the group consisting of: C₁₋₃-alkyl and 6-membered aryl.

In an embodiment, R₁ and R₂ are each independently selected from the group consisting of: C₁₋₂-alkyl and 6-membered aryl.

In an embodiment, R₁ and R₂ are each independently selected from the group consisting of: methyl and 6-membered aryl.

In an embodiment, R₁ and R₂ are each independently selected from the group consisting of: C₁₋₁₅-alkyl and phenyl.

In an embodiment, R₁ and R₂ are each independently selected from the group consisting of: C₁₋₁₂-alkyl and phenyl.

In an embodiment, R₁ and R₂ are each independently selected from the group consisting of: C₁₋₁₀-alkyl and phenyl.

In an embodiment, R₁ and R₂ are each independently selected from the group consisting of: C₁₋₈-alkyl and phenyl.

In an embodiment, R₁ and R₂ are each independently selected from the group consisting of: C₁₋₆-alkyl and phenyl.

In an embodiment, R₁ and R₂ are each independently selected from the group consisting of: C₁₋₄-alkyl and phenyl.

In an embodiment, R₁ and R₂ are each independently selected from the group consisting of: C₁₋₃-alkyl and phenyl.

In an embodiment, R₁ and R₂ are each independently selected from the group consisting of: C₁₋₂-alkyl and phenyl.

In an embodiment, R₁ and R₂ are each independently selected from the group consisting of: methyl and phenyl.

In an embodiment, R₁ and R₂ are each independently a C₁₋₁₅-alkyl.

In an embodiment, R₁ and R₂ are each independently a C₁₋₁₂-alkyl.

In an embodiment, R₁ and R₂ are each independently a C₁₋₁₀-alkyl.

In an embodiment, R₁ and R₂ are each independently a C₁₋₈-alkyl.

In an embodiment, R₁ and R₂ are each independently a C₁₋₆-alkyl.

In an embodiment, R₁ and R₂ are each independently a C₁₋₄-alkyl.

In an embodiment, R₁ and R₂ are each independently a C₁₋₃-alkyl.

In an embodiment, R₁ and R₂ are each independently a C₁₋₂-alkyl.

In an embodiment, R₁ and R₂ are each a methyl group.

In an embodiment, R₁ and R₂, together with the nitrogen atom to which they are attached, form a heterocyclic ring having from 5 to 8 atoms.

It may be that R₁ and R₂ taken together with the nitrogen atom to which they are attached form a heterocyclic ring having from 5 to 7 atoms. It may be that R₁ and R₂ taken together with the nitrogen atom to which they are attached form a heterocyclic ring having from 5 to 6 atoms.

It may be that R₁ and R₂ taken together with the nitrogen atom to which they are attached form a heterocyclic ring having 5 atoms, for example a pyrrolidine ring.

Preferably, R₁ and R₂ taken together with the nitrogen atom to which they are attached form a heterocyclic ring having 6 atoms, for example a piperidine ring.

In an embodiment, m is 1 to 3.

In an embodiment, m is 2 to 3.

In an embodiment, m is 2.

In an embodiment, the initiator component comprises a tertiary amine salt of formula (III):

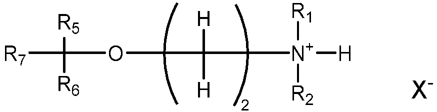

In an embodiment, R₅ and R₆ are each independently selected from the group consisting of: 6 to 10 membered aryl and 5 to 10 membered heteroaryl.

In an embodiment, R₅ and R₆ are each independently selected from the group consisting of: phenyl and 5 to 10 membered heteroaryl.

In an embodiment, R₅ and R₆ are each independently selected from the group consisting of: phenyl and 5 to 9 membered heteroaryl.

In an embodiment, R₅ and R₆ are each independently selected from the group consisting of: phenyl and 5 to 6 membered heteroaryl.

In an embodiment, R₅ and R₆ are each independently selected from the group consisting of: phenyl and pyridinyl (for example, 2-pyridyl, 3-pyridyl, or 4-pyridyl).

In an embodiment R₅ and R₆ are both pyridinyl (for example, 2-pyridyl, 3-pyridyl, or 4-pyridyl).

In an embodiment, R₅ and R₆ are both phenyl.

Any of the alkyl, heterocycloalkyl, aryl or heteroaryl groups of the above R₁ to R₆ groups, can be optionally substituted with from 1 to 3 independently selected groups selected from: halo, C₁₋₄-alkyl or -(CH₂)_{q}-phenyl; wherein q is 0 - 2.

In an embodiment, any of the alkyl, heterocycloalkyl, aryl or heteroaryl groups of the above R₁ to R₆ groups, can be optionally substituted with from 1 to 3 independently selected groups selected from: halo, C₁₋₃-alkyl or -(CH₂)_{q}-phenyl; wherein q is 0 - 2.

In an embodiment, any of the alkyl, heterocycloalkyl, aryl or heteroaryl groups of the above R₁ to R₆ groups, can be optionally substituted with from 1 to 3 independently selected groups selected from: halo, C₁₋₂-alkyl or -(CH₂)_{q}-phenyl; wherein q is 0 - 2.

In an embodiment, any of the alkyl, heterocycloalkyl, aryl or heteroaryl groups of the above R₁ to R₆ groups, can be optionally substituted with from 1 to 3 independently selected groups selected from: halo, C₁-alkyl or -(CH₂)_{q}-phenyl; wherein q is 0 - 2.

In an embodiment, any of the alkyl, heterocycloalkyl, aryl or heteroaryl groups of the above R₁ to R₆ groups, can be optionally substituted with from 1 to 3 independently selected groups selected from: halo, C₁₋₄-alkyl or -(CH₂)_{q}-phenyl; wherein q is 0 - 1.

In an embodiment, any of the alkyl, heterocycloalkyl, aryl or heteroaryl groups of the above R₁ to R₆ groups, can be optionally substituted with from 1 to 3 independently selected groups selected from: halo, C₁₋₃-alkyl or -(CH₂)_{q}-phenyl; wherein q is 0 - 1.

In an embodiment, any of the alkyl, heterocycloalkyl, aryl or heteroaryl groups of the above R₁ to R₆ groups, can be optionally substituted with from 1 to 3 independently selected groups selected from: halo, C₁₋₂-alkyl or -(CH₂)_{q}-phenyl; wherein q is 0 - 1.

In an embodiment, any of the alkyl, heterocycloalkyl, aryl or heteroaryl groups of the above R₁ to R₆ groups, can be optionally substituted with from 1 to 3 independently selected groups selected from: halo, C₁-alkyl or -(CH₂)_{q}-phenyl; wherein q is 0 - 1.

In an embodiment, any of the aryl or heteroaryl groups of the above R₅ or R₆ groups may be substituted with 1 to 3 independently selected halo groups. Any of the aryl or heteroaryl groups of the above R₅ or R₆ groups may be substituted with 1 to 3 chloride groups. Any of the aryl or heteroaryl groups of the above R₅ or R₆ groups may be substituted with 1 to 3 bromide groups. Any of the aryl or heteroaryl groups of the above R₅ or R₆ groups may be substituted with 1 to 3 fluoride groups. Any of the aryl or heteroaryl groups of the above R₅ or R₆ groups may be substituted with 1 to 3 iodide groups.

Any of the aryl or heteroaryl groups of the above R₅ or R₆ groups may be substituted with 1 to 3 C₁₋₄-alkyl groups. Any of the aryl or heteroaryl groups of the above R₅ or R₆ groups may be substituted with 1 to 3 C₁₋₃-alkyl groups. Any of the aryl or heteroaryl groups of the above R₅ or R₆ groups may be substituted with 1 to 3 methyl groups.

In an embodiment, any of the aryl or heteroaryl groups of the above R₅ or R₆ groups may be substituted with 1 to 3 independently selected groups selected from halo or C₁₋₄-alkyl. In an embodiment, any of the aryl or heteroaryl groups of the above R₅ or R₆ groups may be substituted with 1 to 3 independently selected groups selected from halo or C₁₋₃-alkyl. In an embodiment, any of the aryl or heteroaryl groups of the above R₅ or R₆ groups may be substituted with 1 to 3 independently selected groups selected from halo or methyl.

In an embodiment, any of the aryl or heteroaryl groups of the above R₅ or R₆ groups may be substituted with 1 to 3 independently selected groups selected from chloride or C₁₋₄-alkyl. In an embodiment, any of the aryl or heteroaryl groups of the above R₅ or R₆ groups may be substituted with 1 to 3 independently selected groups selected from chloride or C₁₋₃-alkyl. In an embodiment, any of the aryl or heteroaryl groups of the above R₅ or R₆ groups may be substituted with 1 to 3 independently selected groups selected from chloride or methyl.

In an embodiment, any of the aryl or heteroaryl groups of the above R₅ or R₆ groups may be substituted with 1 halo group. Any of the aryl or heteroaryl groups of the above R₅ or R₆ groups may be substituted with 1 chloride group. Any of the aryl or heteroaryl groups of the above R₅ or R₆ groups may be substituted with 1 bromide group. Any of the aryl or heteroaryl groups of the above R₅ or R₆ groups may be substituted with 1 fluoride group. Any of the aryl or heteroaryl groups of the above R₅ or R₆ groups may be substituted with 1 iodide group.

Any of the aryl or heteroaryl groups of the above R₅ or R₆ groups may be substituted with 1 C₁₋₄-alkyl group. Any of the aryl or heteroaryl groups of the above R₅ or R₆ groups may be substituted with 1 C₁₋₃-alkyl group. Any of the aryl or heteroaryl groups of the above R₅ or R₆ groups may be substituted with 1 methyl group.

In an embodiment, any of the aryl or heteroaryl groups of the above R₅ or R₆ groups may be substituted with 1 group selected from halo or C₁₋₄-alkyl. In an embodiment, any of the aryl or heteroaryl groups of the above R₅ or R₆ groups may be substituted with 1 group selected from halo or C₁₋₃-alkyl. In an embodiment, any of the aryl or heteroaryl groups of the above R₅ or R₆ groups may be substituted with 1 group selected from halo or methyl.

In an embodiment, any of the aryl or heteroaryl groups of the above R₅ or R₆ groups may be substituted with 1 group selected from chloride or C₁₋₄-alkyl. In an embodiment, any of the aryl or heteroaryl groups of the above R₅ or R₆ groups may be substituted with 1 group selected from chloride or C₁₋₃-alkyl. In an embodiment, any of the aryl or heteroaryl groups of the above R₅ or R₆ groups may be substituted with 1 group selected from chloride or methyl.

In some embodiments, at least one of R₅ or R₆ may be phenyl substituted with a methyl group. In some embodiments, at least one of R₅ or R₆ may be phenyl substituted with a chloride group. In some embodiments, R₅ is an unsubstituted phenyl and R₆ is a phenyl substituted with a chloride group. In some embodiments, R₅ is an unsubstituted phenyl and R₆ is a phenyl substituted with a methyl group.

In some embodiments, at least one of R₅ or R₆ may be pyridinyl substituted with a methyl group. In some embodiments, at least one of R₅ or R₆ may be pyridinyl substituted with a chloride group. In some embodiments, R₅ is an unsubstituted pyridinyl and R₆ is a pyridinyl substituted with a chloride group. In some embodiments, R₅ is an unsubstituted pyridinyl and R₆ is a pyridinyl substituted with a methyl group.

In some embodiments, R₅ is an unsubstituted phenyl and R₆ is a pyridinyl substituted with a chloride group. In some embodiments, R₅ is an unsubstituted phenyl and R₆ is a pyridinyl substituted with a methyl group. In an embodiment, R₅ is an unsubstituted pyridinyl and R₆ is a phenyl substituted with a chloride group. In some embodiments, R₅ is an unsubstituted pyridinyl and R₆ is a phenyl substituted with a methyl group.

In an embodiment, R₇ is selected from the group consisting of: H and C₁-₁₂-alkyl. In an embodiment, R₇ is selected from the group consisting of: H and C₁-₁₀-alkyl. In an embodiment, R₇ is selected from the group consisting of: H and C₁-₈-alkyl. In an embodiment, R₇ is selected from the group consisting of: H and, C₁-₆-alkyl. In an embodiment, R₇ is selected from the group consisting of: H and C₁-₄-alkyl. In an embodiment, R₇ is selected from the group consisting of: H and C₁-₃-alkyl.

In an embodiment, R₇ is H.

In an embodiment, R₇ is C₁₋₁₂-alkyl.

In an embodiment, R₇ is C₁₋₁₀-alkyl.

In an embodiment, R₇ is C₁₋₈-alkyl.

In an embodiment, R₇ is C₁₋₆-alkyl.

In an embodiment, R₇ is C₁₋₄-alkyl.

In an embodiment, R₇ is C₁₋₃-alkyl, e.g. methyl, ethyl or propyl.

In an embodiment, X⁻ is selected from halide, maleate, acetate, sulfate, hydrogen sulfate, cyano, chloroacetate, cyanoacetate or succinate.

In an embodiment, X⁻ is selected from halide, maleate, acetate, sulfate, hydrogen sulfate, cyano, chloroacetate, cyanoacetate.

In an embodiment, X⁻ is selected from halide, maleate, acetate or succinate.

In an embodiment, X⁻ is selected from halide, maleate, acetate.

In an embodiment, X⁻ is selected from Cl⁻, F⁻, Br, I⁻, maleate, acetate or succinate.

In an embodiment, X⁻ is selected from Cl⁻, F⁻, Br, I⁻, maleate, acetate.

In an embodiment, R₁ and R₂ are each independently a C₁₋₄-alkyl, and m is 1 to 3. Preferably, R₁ and R₂ are each independently a C₁₋₄-alkyl, and m is 2 to 3.

In an embodiment, R₁ and R₂ are each independently a C₁₋₃-alkyl, and m is 1 to 3. Preferably, R₁ and R₂ are each independently a C₁₋₃-alkyl, and m is 2 to 3.

In an embodiment, R₁ and R₂ are each independently a C₁₋₂-alkyl, and m is 1 to 3. Preferably, R₁ and R₂ are each independently a C₁₋₂-alkyl, and m is 2 to 3.

In an embodiment, R₁ and R₂ are each a methyl group, and m is 1 to 3. Preferably, R₁ and R₂ are each a methyl group, and m is 2 to 3.

In an embodiment, R₁ and R₂ are each independently a C₁₋₄-alkyl, and R₅ and R₆ are each phenyl and 5 to 6 membered heteroaryl. In an embodiment, R₁ and R₂ are each independently a C₁₋₄-alkyl, m is 1 to 3, and R₅ and R₆ are each phenyl and 5 to 6 membered heteroaryl. Preferably, R₁ and R₂ are each independently a C₁₋₄-alkyl, m is 2 to 3, and R₅ and R₆ are each phenyl and 5 to 6 membered heteroaryl.

In an embodiment, R₁ and R₂ are each independently a C₁₋₃-alkyl, and R₅ and R₆ are each phenyl and 5 to 6 membered heteroaryl. In an embodiment, R₁ and R₂ are each independently a C₁₋₃-alkyl, m is 1 to 3, and R₅ and R₆ are each phenyl and 5 to 6 membered heteroaryl. Preferably, R₁ and R₂ are each independently a C₁₋₃-alkyl, m is 2 to 3, and R₅ and R₆ are each phenyl and 5 to 6 membered heteroaryl.

In an embodiment, R₁ and R₂ are each independently a C₁₋₂-alkyl, and R₅ and R₆ are each phenyl and 5 to 6 membered heteroaryl. In an embodiment, R₁ and R₂ are each independently a C₁₋₂-alkyl, m is 1 to 3, and R₅ and R₆ are each phenyl and 5 to 6 membered heteroaryl. Preferably, R₁ and R₂ are each independently a C₁₋₂-alkyl, m is 2 to 3, and R₅ and R₆ are each phenyl and 5 to 6 membered heteroaryl.

In an embodiment, R₁ and R₂ are each a methyl group, and R₅ and R₆ are each phenyl and 5 to 6 membered heteroaryl. In an embodiment, R₁ and R₂ are each a methyl group, m is 1 to 3, and R₅ and R₆ are each H, phenyl and 5 to 6 membered heteroaryl. Preferably, R₁ and R₂ are each a methyl group, m is 2 to 3, and R₅ and R₆ are each phenyl and 5 to 6 membered heteroaryl.

In an embodiment, R₁ and R₂ are each independently a C₁₋₄-alkyl, m is 1 to 3, R₅ and R₆ are each phenyl and 5 to 6 membered heteroaryl and R₇ is independently selected from the group consisting of: H and C₁₋₁₂-alkyl. Preferably, R₁ and R₂ are each independently a C₁₋₄-alkyl, m is 2 to 3, R₅ and R₆ are each phenyl and 5 to 6 membered heteroaryl and R₇ is independently selected from the group consisting of: H and C₁₋₁₂-alkyl.

In an embodiment, R₁ and R₂ are each independently a C₁₋₃-alkyl, m is 1 to 3, R₅ and R₆ are each phenyl and 5 to 6 membered heteroaryl and R₇ is independently selected from the group consisting of: H and C₁₋₁₂-alkyl. Preferably, R₁ and R₂ are each independently a C₁₋₃-alkyl, m is 2 to 3, R₅ and R₆ are each phenyl and 5 to 6 membered heteroaryl and R₇ is independently selected from the group consisting of: H and C₁₋₁₂-alkyl.

In an embodiment, R₁ and R₂ are each independently a C₁₋₂-alkyl, m is 1 to 3, R₅ and R₆ are each phenyl and 5 to 6 membered heteroaryl and R₇ is independently selected from the group consisting of: H and C₁₋₁₂-alkyl. Preferably, R₁ and R₂ are each independently a C₁₋₂-alkyl, m is 2 to 3, R₅ and R₆ are each phenyl and 5 to 6 membered heteroaryl and R₇ is independently selected from the group consisting of: H and C₁₋₁₂-alkyl.

In an embodiment, R₁ and R₂ are each a methyl group, m is 1 to 3, R₅ and R₆ are each phenyl and 5 to 6 membered heteroaryl and R₇ is independently selected from the group consisting of: H and C₁₋₁₂-alkyl. Preferably, R₁ and R₂ are each a methyl group, m is 2 to 3, R₅ and R₆ are each phenyl and 5 to 6 membered heteroaryl and R₇ is independently selected from the group consisting of: H and C₁₋₁₂-alkyl.

In an embodiment m is 1 to 3, and R₅ and R₆ are both phenyl. In an embodiment m is 2 to 3, and R₅ and R₆ are both phenyl.

In an embodiment R₅ and R₆ are both phenyl and R₇ is H. In an embodiment m is 1 to 3, R₅ and R₆ are both phenyl and R₇ is H. In an embodiment m is 2 to 3, R₅ and R₆ are both phenyl and R₇ is H.

In an embodiment m is 1 to 3, and R₅ and R₆ are selected from phenyl and pyridinyl. In an embodiment m is 2 to 3, and R₅ and R₆ are selected from phenyl and pyridinyl.

In an embodiment R₅ and R₆ are selected from phenyl and pyridinyl and R₇ is H. In an embodiment m is 1 to 3, R₅ and R₆ are selected from phenyl and pyridinyl and R₇ is H. In an embodiment m is 2 to 3, R₅ and R₆ are selected from phenyl and pyridinyl and R₇ is H.

In an embodiment, the tertiary amine salt is selected from the group consisting of: 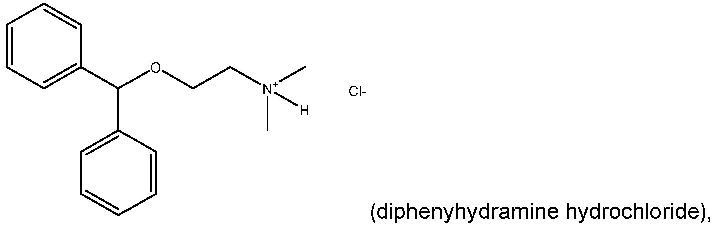 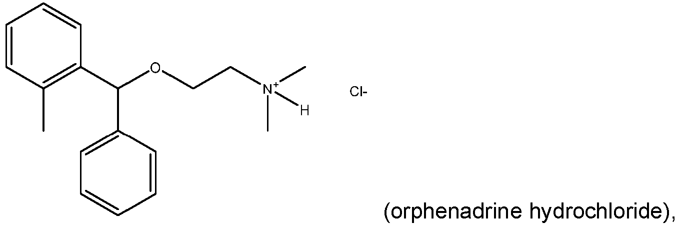 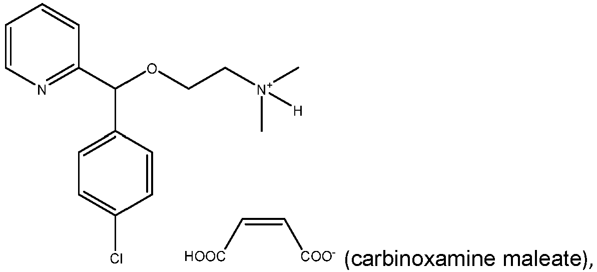 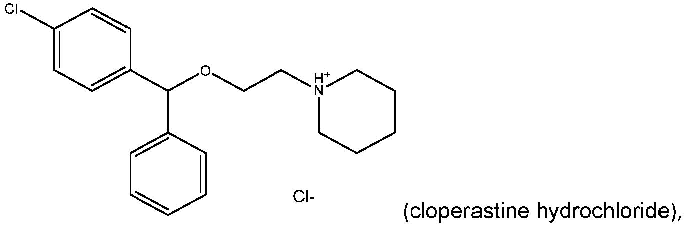 and

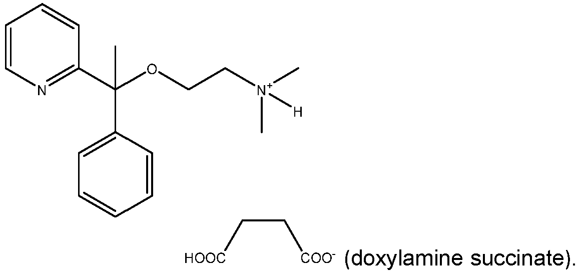

In an embodiment, the kit comprises a mixture diphenyhydramine hydrochloride and at least one other tertiary amine salt selected from the group consisting of: orphenadrine hydrochloride, carbinoxamine maleate, cloperastine hydrochloride and doxylamine succinate. For example, the kit may comprise a mixture of diphenyhydramine hydrochloride and cloperastine hydrochloride. The kit may comprise a mixture of diphenyhydramine hydrochloride and orphenadrine hydrochloride. The kit may comprise a mixture of diphenyhydramine hydrochloride and carbinoxamine maleate. The kit may comprise a mixture of diphenyhydramine hydrochloride and doxylamine succinate.

The mole ratio of diphenyhydramine hydrochloride to the at least one other tertiary amine salt may be in the range of from about 1:10 to about 10:1. The mole ratio of diphenyhydramine hydrochloride to the at least one other tertiary amine salt may be in the range of from about 1:9 to about 10:1. The mole ratio of diphenyhydramine hydrochloride to the at least one other tertiary amine salt may be in the range of from about 1:8 to about 10:1. The mole ratio of diphenyhydramine hydrochloride to the at least one other tertiary amine salt may be in the range of from about 1:7 to about 10:1. The mole ratio of diphenyhydramine hydrochloride to the at least one other tertiary amine salt may be in the range of from about 1:6 to about 10:1. The mole ratio of diphenyhydramine hydrochloride to the at least one other tertiary amine salt may be in the range of from about 1:5 to about 10:1. The mole ratio of diphenyhydramine hydrochloride to the at least one other tertiary amine salt may be in the range of from about 1:4 to about 10:1. The mole ratio of diphenyhydramine hydrochloride to the at least one other tertiary amine salt may be in the range of from about 1:3 to about 10:1. The mole ratio of diphenyhydramine hydrochloride to the at least one other tertiary amine salt may be in the range of from about 1:2 to about 10:1. The mole ratio of diphenyhydramine hydrochloride to the at least one other tertiary amine salt may be in the range of from about 1:1 to about 10:1.

The mole ratio of diphenyhydramine hydrochloride to cloperastine hydrochloride may be in the range of from about 1:10 to about 10:1. The mole ratio of diphenyhydramine hydrochloride to cloperastine hydrochloride may be in the range of from about 1:9 to about 10:1. The mole ratio of diphenyhydramine hydrochloride to cloperastine hydrochloride may be in the range of from about 1:8 to about 10:1. The mole ratio of diphenyhydramine hydrochloride to cloperastine hydrochloride may be in the range of from about 1:7 to about 10:1. The mole ratio of diphenyhydramine hydrochloride to cloperastine hydrochloride may be in the range of from about 1:6 to about 10:1. The mole ratio of diphenyhydramine hydrochloride to cloperastine hydrochloride may be in the range of from about 1:5 to about 10:1. The mole ratio of diphenyhydramine hydrochloride to cloperastine hydrochloride may be in the range of from about 1:4 to about 10:1. The mole ratio of diphenyhydramine hydrochloride to cloperastine hydrochloride may be in the range of from about 1:3 to about 10:1. The mole ratio of diphenyhydramine hydrochloride to cloperastine hydrochloride may be in the range of from about 1:2 to about 10:1. The mole ratio of diphenyhydramine hydrochloride to cloperastine hydrochloride may be in the range of from about 1:1 to about 10:1.

The mole ratio of diphenyhydramine hydrochloride to the at least one other tertiary amine salt may be in the range of from about 1:5 to about 5:1. The mole ratio of diphenyhydramine hydrochloride to the at least one other tertiary amine salt may be in the range of from about 1:4 to about 4:1. The mole ratio of diphenyhydramine hydrochloride to the at least one other tertiary amine salt may be in the range of from about 1:3 to about 3:1. The mole ratio of diphenyhydramine hydrochloride to the at least one other tertiary amine salt may be in the range of from about 1:2 to about 2:1. The mole ratio of diphenyhydramine hydrochloride to the at least one other tertiary amine salt may be in the range of from about 1:1 to about 1:1.

The mole ratio of diphenyhydramine hydrochloride to cloperastine hydrochloride may be in the range of from about 1:5 to about 5:1. The mole ratio of diphenyhydramine hydrochloride to cloperastine hydrochloride may be in the range of from about 1:4 to about 4:1. The mole ratio of diphenyhydramine hydrochloride to cloperastine hydrochloride may be in the range of from about 1:3 to about 3:1. The mole ratio of diphenyhydramine hydrochloride to cloperastine hydrochloride may be in the range of from about 1:2 to about 2:1. The mole ratio of diphenyhydramine hydrochloride to cloperastine hydrochloride may be in the range of from about 1:1 to about 1:1.

In an embodiment, the cyanoacrylate component is defined as in US 8,475,825, columns 6-8.

In an embodiment, the cyanoacrylate component has formula (IV): 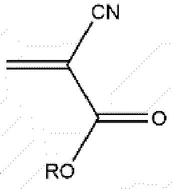 wherein R is C₁-C₂₀ alkyl, C₂-C₂₀ alkenyl or C₂-C₂₀ alkynyl, wherein R is optionally substituted with one or more moieties selected from the group consisting of: -O-C₁-C₁₀ alkyl, -C(O)O-C₁-C₁₀ alkyl, aryl and heteroaryl, wherein any of the alkyl, alkenyl, alkynyl, aryl or heteroaryl groups can be optionally substituted with from 1 to 6 independently selected halo groups.

In an embodiment, R is a C₁-C₁₅ alkyl, C₂-C₁₅ alkenyl or a C₂-C₁₅ alkynyl. In an embodiment, R is a C₁-C₁₀ alkyl, C₂-C₁₀ alkenyl or a C₂-C₁₀ alkynyl. In an embodiment, R is a C₁-C₈ alkyl, C₂-C₈ alkenyl or a C₂-C₈ alkynyl.

In an embodiment, R is a C₁-C₂₀ alkyl. In an embodiment, R is a C₁-C₁₅ alkyl. In an embodiment, R is a C₁-C₁₀ alkyl. In an embodiment, R is a C₁-C₈ alkyl.

In an embodiment, the cyanoacrylate component is selected from 2-octylcyanoacrylate, n-2-butyl-cyanoacrylate or 2-ethylcyanoacrylate. In a preferred embodiment, the cyanoacrylate component is 2-octylcyanoacrylate.

In an embodiment, R is a C₂-C₂₀ alkenyl. In an embodiment, R is a C₂-C₁₅ alkenyl. In an embodiment, R is a C₂-C₁₀ alkenyl. In an embodiment, R is a C₂-C₈ alkenyl.

In an embodiment, R is a C₂-C₂₀ alkynyl. In an embodiment, R is a C₂-C₁₅ alkynyl. In an embodiment, R is a C₂-C₁₀ alkynyl. In an embodiment, R is a C₂-C₈ alkynyl.

In an embodiment, R is substituted with one or more moieties selected from the group consisting of an -O-C₁-C₈ alkyl, -C(O)O-C₁-C₈ alkyl, aryl and heteroaryl. In an embodiment, R is substituted with one or more moieties selected from the group consisting of an -O-C₁-C₆ alkyl, -C(O)O-C₁-C₆ alkyl, aryl and heteroaryl. In an embodiment, R is substituted with one or more moieties selected from the group consisting of an -O-C₁-C₄ alkyl, -C(O)O-C₁-C₄ alkyl, aryl and heteroaryl. In an embodiment, R is substituted with one or more moieties selected from the group consisting of an -O-C₁-C₂ alkyl, -C(O)O-C₁-C₂ alkyl, aryl and heteroaryl.

In an embodiment, R is substituted with an ether moiety, -O-C₁-C₁₀ alkyl. In an embodiment, R is substituted with an ether moiety, -O-C₁-C₈ alkyl. In an embodiment, R is substituted with an ether moiety, -O-C₁-C₆ alkyl. In an embodiment, R is substituted with an ether moiety, -O-C₁-C₄ alkyl. In an embodiment, R is substituted with an ether moiety, -O-C₁-C₂ alkyl.

In an embodiment, R is substituted with an ester moiety C(O)O-C₁-C₁₀ alkyl. In an embodiment, R is substituted with an ester moiety C(O)O-C₁-C₈ alkyl. In an embodiment, R is substituted with an ester moiety C(O)O-C₁-C₆ alkyl. In an embodiment, R is substituted with an ester moiety C(O)O-C₁-C₄ alkyl. In an embodiment, R is substituted with an ester moiety C(O)O-C₁-C₄ alkyl.

In an embodiment, R is substituted with an aryl moiety, optionally a phenyl moiety.

In an embodiment, R is substituted with an heteroaryl moiety.

In an embodiment, R, or any of its substituents thereon, is further substituted with 1 to 3 halide groups. In an embodiment, R, or any of its substituents thereon, is further substituted with 1 to 2 halide groups. In an embodiment, R, or any of its substituents thereon, is further substituted with 1 halide group. In an embodiment, R, or any of its substituents thereon, is further substituted with 1 to 3 fluoride groups. In an embodiment, R, or any of its substituents thereon, is further substituted with 1 to 2 fluoride groups. In an embodiment, R, or any of its substituents thereon, is further substituted with 1 fluoride group. In an embodiment, R, or any of its substituents thereon, is further substituted with 1 to 3 chloride groups. In an embodiment, R, or any of its substituents thereon, is further substituted with 1 to 2 chloride groups. In an embodiment, R, or any of its substituents thereon, is further substituted with 1 chloride group.

In an embodiment the cyanoacrylate component is selected from n-octyl cyanoacrylate, n-hexyl cyanoacrylate, adamantly cyanoacrylate, ethyl cyanoacrylate, 2-octyl cyanoacrylate, dodecyl cyanoacrylate, 2-ethylhexyl cyanoacrylate, butyl cyanoacrylate, methyl cyanoacrylate, 3-methoxylbutyl cyanoacrylate, 2-butoxylethyl cyanoacrylate, 2-isopropyoxylethyl cyanoacrylate, or 1-methoxyl-2-propyl cyanoacrylate. Preferably, the cyanoacrylate monomer is 2-octyl cyanoacrylate, abbreviated 2-OCA.

In an embodiment, the cyanoacrylate component is present in the adhesive component from about 50% to about 100% by weight, for example from about 75% to about 99%, from about 80% to about 96%, from about 85% to about 94% by weight.

In an embodiment, the initiator component and/or the adhesive component further comprises an accelerant or a curing rate modifier. In an embodiment, the initiator component further comprises an accelerant or a curing rate modifier. In an embodiment, the adhesive component further comprises an accelerant or a curing rate modifier.

In an embodiment, the accelerant or a curing rate modifier is selected from the group consisting of: crown ether, polyalkylene oxide, silacrown ether, calixarene, cyclodextrin and progallol-based cyclic compounds. In an embodiment the accelerant or a curing rate modifier is a crown ether. In an embodiment the accelerant or a curing rate modifier is a crown ether selected from the group consisting of: 12-crown-4, 15-crown-5, 18-crown-6, benzo-12-crown-4, benzo-15-crown-5, benzo-18-crown-6, dibenzo-18-crown-6, dibenzo-24-crown-8, dibenzo-30-crown-10, tribenzo-18-crown-6, asym-dibenzo-22-crown-6, dibenzo-14-crown-4, dicyclohexyl-24-crown-8, cyclohexyl-12-crown-4, 1,2-decalyl-15-crown-5, 1,2-naphtho-15-crown-5, 3,4,5-naphthyl-16-crown-5, 1,2-methylbenzo-18-crown-6, 1,2-tert-butyl-18-crown-6, and 1,2-vinylbenzo-15-crown-5. In a preferred embodiment, the accelerant or a curing rate modifier is 18-crown-6.

In a preferred embodiment, the initiator component and/or the adhesive component does not include an accelerant or a curing rate modifier. In an embodiment, the initiator component does not include an accelerant or a curing rate modifier. In an embodiment, the adhesive component does not include an accelerant or a curing rate modifier.

In an embodiment, the adhesive component further comprises a thickener. Examples of the thickener include poly(alkyl methacrylate) (e.g. poly(2-ethylhexyl methacrylate, polymethylmethacrylate), a poly(alkyl acrylate) (e.g. poly(2-ethylhexyl acrylate), a poly(cyanoacrylate) (e.g. poly(2-octyl cyanoacrylate), a poly(oxalate), poly (lactic acid), poly (glycolic acid), a lactic-glycolic copolymer, a polycaprolactone, a lactic acid-caprolactone copolymer, a polyvinyl acetate, a copolymer of polyacrylate or methacrylate and butadiene, or a polyorthoester. The thickener may include preformed polymers of cyanoacrylates, polymethylmethacrylate, poly (lactic acid), poly (glycolic acid), polycaprolactone and copolymers.

When a thickener is included, the thickener is present from about 0% to about 20% by weight, for example from about 0% to about 10% by weight. Alternatively, a thickener need not be present.

In an embodiment, the adhesive component further comprises a plasticizing agent. Examples of plasticizing agents include acetal trihexyl citrate, cetyl trihexyl citrate, fatty acid esters, tributyl citrate, acetyl tri-n-butyl citrate (ATBC), polymethylmethacrylate, polydimethylsiloxane, hexadimethylsilazane, dibutyl sebacate, or tributyl O-acetyl citrate.

Examples of plasticizing agents include acetal trihexyl citrate, cetyl trihexyl citrate, fatty acid esters, tributyl citrate, acetyl tri-n-butyl citrate (ATBC), polymethylmethacrylate, polydimethylsiloxane, hexadimethylsilazane,

When a plasticizer is included, the plasticizer is present from about 0% to about 25% by weight, for example from about 0% to about 10% by weight. Alternatively, a plasticiser need not be present.

In an embodiment, the adhesive component further comprises a stabilizer. Examples of the stabiliser may be butylated hydroxyl anisole or sulfur dioxide. When a stabiliser is included, the stabiliser is present from about 0 to about 5000 ppm, for example from about 100ppm to about 1500ppm.

In an embodiment, the initiator component comprises a solid substrate, and wherein the tertiary amine salt is deposited onto and/or into the solid substrate.

In an embodiment, the solid substrate is made of polyurethane, polyesters, polyolefins such as polyethylene, or polyamides.

In an embodiment, the solid substrate is made of polyethylene. For example, the solid substrate may be high density polyethylene (HDPE).

In an embodiment, the solid substrate is a sintered porous plastic filter, made of high density polyethylene (HDPE).

In an embodiment, solid substrate is a porous solid substrate and the tertiary amine salt is deposited into the solid substrate, i.e. impregnated into the pores of the porous solid substrate. It may be that a mixture of more than one tertiary amine salt described herein is deposited onto and/or into the porous solid substrate. For example, a mixture of two tertiary amine salts may be deposited onto and/or into the porous solid substrate. For example, a mixture of three tertiary amine salts may be deposited onto and/or into the porous solid substrate.

An exemplary material that can be used as the porous solid substrate is a sintered porous HDPE filter obtainable from Porex Technologies Corp.

In an embodiment, solid substrate is a non-porous solid substrate and tertiary amine salt is deposited onto the solid substrate.

In an embodiment the tertiary amine salt is present on and/or in the porous substrate at a quantity of 0.1 to 20*10⁻³ mmol. In an embodiment the tertiary amine salt is present in the porous substrate at a quantity of 0.5 to 20*10⁻³ mmol. In an embodiment the tertiary amine salt is present in the porous substrate at a quantity of 1 to 20*10⁻³ mmol. In an embodiment the tertiary amine salt is present in the porous substrate at a quantity of 0.1 to 15*10⁻³ mmol. In an embodiment the tertiary amine salt is present in the porous substrate at a quantity of 0.1 to 10*10⁻³ mmol. In an embodiment the tertiary amine salt is present in the porous substrate at a quantity of 0.1 to 5*10⁻³ mmol. In an embodiment the tertiary amine salt is present in the porous substrate at a quantity of 0.5 to 10*10⁻³ mmol. In an embodiment the tertiary amine salt is present in the porous substrate at a quantity of 1 to 5*10⁻³ mmol.

In an embodiment the tertiary amine salt is present at from 50 to 5000 ppm adhesive component. In an embodiment the tertiary amine salt is present at from 100 to 2500 ppm adhesive component (e.g. 500 to 1000 ppm). In an embodiment the tertiary amine salt is present at from 100 to 2000 ppm adhesive component. In an embodiment the tertiary amine salt is present at from 150 to 1500 ppm adhesive component. In an embodiment the tertiary amine salt is present at from 200 to 1200 ppm adhesive component. In an embodiment, the tertiary amine salt is present at from 400 to 1200 ppm adhesive component. In an embodiment, the tertiary amine salt is present at from 250 to 1000 ppm adhesive component. In an embodiment, the tertiary amine salt is present at from 250 to 800 ppm adhesive component. In an embodiment, the tertiary amine salt is present at from 250 to 650 ppm adhesive component.

In an embodiment a mixture of more than one tertiary amine salt is present at from 50 to 5000 ppm adhesive component. In an embodiment a mixture of more than one tertiary amine salt is present at from 100 to 2500 ppm adhesive component (e.g. 500 to 1000 ppm). In an embodiment a mixture of more than one tertiary amine salt is present at from 100 to 2000 ppm adhesive component. In an embodiment a mixture of more than one tertiary amine salt is present at from 150 to 1500 ppm adhesive component. In an embodiment a mixture of more than one tertiary amine salt is present at from 200 to 1200 ppm adhesive component. In an embodiment, a mixture of more than one tertiary amine salt is present at from 400 to 1200 ppm adhesive component. In an embodiment, a mixture of more than one tertiary amine salt is present at from 250 to 1000 ppm adhesive component. In an embodiment, a mixture of more than one tertiary amine salt is present at from 250 to 800 ppm adhesive component. In an embodiment, a mixture of more than one tertiary amine salt is present at from 250 to 650 ppm adhesive component.

According to a second aspect, there is provided a wound closure adhesive composition comprising:
a cyanoacrylate; and
a tertiary amine salt;
wherein the cyanoacrylate and tertiary amine salt are as defined according the first aspect.

According to a third aspect, there is provided a use of a tertiary amine salt as an initiator in a cyanoacrylate wound closure adhesive wherein the cyanoacrylate adhesive and tertiary amine salt are as defined according the first aspect.

According to a fourth aspect, there is provided an adhesive composition comprising a cyanoacrylate adhesive and a tertiary amine salt for use in a method of applying a cyanoacrylate wound closure adhesive comprising:
contacting the cyanoacrylate adhesive with a tertiary amine salt;
depositing the cyanoacrylate wound closure adhesive on a surface;
wherein the cyanoacrylate adhesive and tertiary amine salt are as defined according the first aspect.

According to a fifth aspect, there is provided a wound closure adhesive product comprising: a sealed first vessel (e.g. a sealed tube) containing the cyanoacrylate adhesive component (A); and a second vessel (e.g. a connector) containing the initiator component (B), wherein the cyanoacrylate adhesive component (A) and initiator component (B) are as defined according the first aspect. As mentioned above, the initiator component (B) can comprise a solid substrate and the tertiary amine salt is deposited onto and/or into the solid substrate, and the solid substrate can be contained within the second vessel (e.g. a connector).

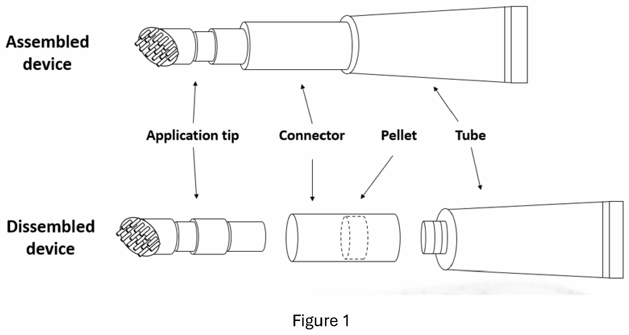

In use, as illustrated in Figure 1, the second vessel (e.g. the connector) with applicator tip inserted, which contains the porous solid substrate (i.e., pellet) impregnated with said initiator(s), is connected to the first vessel (e.g. the sealed tube), which contains the adhesive component. The connection of the first vessel and the second vessel opens the sealed first vessel and exposes the adhesive component. The connection of the first and second vessel allows the adhesive component (A) and the initiator component (B) to contact one another thereby initiating the polymerisation of the adhesive component. For example, once the first and second vessels are connected, the adhesive component can flow through the pellet and contact with the initiator component, e.g. by squeezing the first vessel to force the adhesive component out of the first vessel and then contact the initiator component. Following contact of the adhesive component and the initiator component and/or initiation of the polymerisation of the adhesive component, the resulting adhesive composition can be deposited onto a surface such as human skin. The adhesive deposited on human skin can be cured into a plastic film within a few minutes and stay 7-14 days.

### Description of the figures:

Figure 1 - Sketch of cyanoacrylate topical skin adhesive kit.

### Examples

### Chemical names, abbreviations and structures:

| | |
|---|---|
| **BAK** (Benzalkonium chloride) | **CE** (18-crown-6) |
| 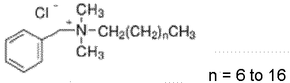 | 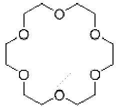 |
| **DPHA** (diphenhydramine) | **DPHA.HCl** (diphenhydramine hydrochloride) |
| 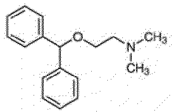 | 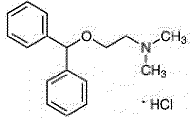 |

The purpose of comparative examples 1 and 2 and example 1 are to demonstrate the curing time of several adhesive compositions when presented as a kit comprising a cyanoacrylate adhesive component and the initiator component as deposited in or on a solid substrate (pellet) in line with the commercial product "Chemence Exofin HVTSA". The porous pellet is as described above, i.e. a sintered porous plastic filter, made from high density polyethylene (HDPE) (for example a sintered porous HDPE filter obtainable from Porex Technologies Corp).

### Comparative Example 1: BAK control (with accelerant)

This example demonstrates that known acyclic quaternary ammonium salts, which are also known to cause allergic contact dermatitis (ACD), especially BAK, can operate as reactive initiators for use in wound closure adhesives compositions.

The BAK and CE were dissolved in IPA to form a stock solution (5.3% BAK and 6.6% CE in IPA) and 15mg of this stock solution was added to a porous pellet to allow the porous pellet to be impregnated with BAK and CE. The IPA was then allowed to evaporate for at least 24 hours at room temperature, resulting in a pellet containing 0.80 mg BAK (2.2*10⁻³ mmol) and 1.0 mg CE.

The porous pellet was inserted into a connector tube as shown in Figure 1 having a threaded bore at one end and an opening at the opposite end.

A container including the 2-cyanoacrylate adhesive formulation (exofin^{®} High Viscosity Topical Skin Adhesive, HVTSA, containing 1 g 2-octylcyanoacrylate, also containing a thickener to control the viscosity; a plasticizing agent that imparts the flexibility; suitable additives and stabilizers) was connected to the threaded bore of the connector tube, the HVTSA was squeezed through the connector tube to allow the 2-cyanoacrylate to come into contact with the initiator and then exit the connector tube through the opening and be deposited onto the surface of a nitrile substrate (0.02 g - 0.05 g).

The curing time was determined from applying the liquid adhesive on the nitrile substrate to the time that the adhesive was solidified into a tacky free polymeric film, t_{c} = 20 (± 2) seconds.

### Comparative example 2: DPHA control (without accelerant)

This comparative example demonstrates the very poor reactivity and slow curing time of a cyanoacrylate adhesive kit comprising the tertiary amine DPHA, wherein the amine is not in salt form.

DPHA was dissolved in IPA to form a 4.32 wt% stock solution. 15 mg of the stock solution was directly dosed onto the surface of a porous pellet and the solvent was allowed to evaporate in air for at least 24 h. The theoretical amount of DPHA initiator in the pellet was 0.65 mg (2.5*10⁻³ mmol).

The treated porous pellet was inserted into a connector tube as shown in Figure 1 having a threaded bore at one end and an opening at the opposite end.

A container including the 2-cyanoacrylate adhesive formulation (exofin^{®} High Viscosity Topical Skin Adhesive, HVTSA, containing 1 g 2-octylcyanoacrylate, also containing a thickener to control the viscosity; a plasticizing agent that imparts the flexibility; suitable additives and stabilizers) was connected to the threaded bore of the connector tube, the HVTSA was squeezed through the connector tube to allow the 2-cyanoacrylate to come into contact with the initiator and then exit the connector tube through the opening and be deposited onto the surface of a nitrile substrate (0.02 g - 0.05 g).

The curing time was determined from applying the liquid adhesive on the nitrile substrate to the time that the adhesive was solidified into a tacky free polymeric film. However, the adhesive was not cured after 30 minutes.

### Example 1: DPHA.HCl

This example demonstrates that the reactivity and curing time of cyanoacrylate adhesive kit comprising DPHA.HCl as the initiator is comparable to BAK (with accelerant) and significantly better than DPHA in Comparative Examples 1 and 2 respectively. Additionally, the curing time is controllable depending on the quantity of initiator deposited in the porous pellet. Tertiary amine salt initiators such as DPHA.HCl are less toxic compared with known acyclic quaternary ammonium salts and are therefore expected to reduce the risk of allergic contact dermatitis (ACD).

Different amounts of DPHA.HCl were dissolved in IPA to give stock solutions with three concentrations. 15 mg of the stock solution was directly dosed onto the surface of a porous pellet and the solvent was allowed to evaporate in air for at least 24 h. The theoretical amount of DPHA.HCl initiator in the pellet was:
(a) 0.65 mg (2.2*10⁻³ mmol).
(b) 0.33 mg (1.1*10⁻³ mmol).
(c) 1.0 mg (3.4*10⁻³ mmol).

The treated porous pellet, with the three varying quantities of DPHA.HCl (a - c), was inserted into a connector tube as shown in Figure 1 having a threaded bore at one end and an opening at the opposite end.

A container including the 2-cyanoacrylate adhesive formulation (exofin^{®} High Viscosity Topical Skin Adhesive, HVTSA, containing 1 g 2-octylcyanoacrylate, also containing a thickener to control the viscosity; a plasticizing agent that imparts the flexibility; suitable additives and stabilizers) was connected to the threaded bore of the connector tube, the HVTSA was squeezed through the connector tube to allow the 2-cyanoacrylate to come into contact with the initiator and then exit the connector tube through the opening and be deposited onto the surface of a thin nitrile substrate (0.02 g - 0.05 g).

The curing time was determined from applying the liquid adhesive on the nitrile substrate to the time that the adhesive was solidified into a tacky free polymeric film, t_{c}. The results are summarised in Table 1 below.

**Table 1.**

| | **Initiator** | **Accelerator (amount)** | **Wt of initiator in pellet (mg)** | **Moles of initiator in pellet (mmol)** | **Curing time, t_{c} (seconds)** |
|---|---|---|---|---|---|
| **Comparative Example 1** | BAK | CE (1.0mg) | 0.80 | 2.2*10⁻³ | **20 ± 2** |
| **Comparative Example 2** | DPHA | - | 0.65 | 2.5*10⁻³ | **Not Cured** |
| **Example 1 (a)** | DPHA.HCl | - | 0.65 | 2.2*10-3 | **44 ± 5** |
| **Example 1 (b)** | DPHA.HCl | - | 0.33 | 1.1*10⁻³ | **59 ± 5** |
| **Example 1 (c)** | DPHA.HCl | - | 1.00 | 3.3*10⁻³ | **35 ± 2** |

| | | | | | |
|---|---|---|---|---|---|
| (N=3 for each test) | | | | | |

### Examples 2 to 8

### Chemical names, abbreviations and structures:

**Table 2, chemical profile**

| Chemical name | Abbreviation | CAS number | Chemical structure |
|---|---|---|---|
| Benzalkonium chloride | BAC | 63449-41-2 | 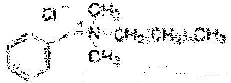 |
| Diphenhydramine hydrochloride | DPHA | 147-24-0 | 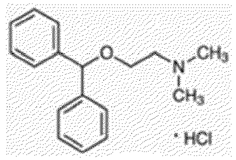 |
| Cloperastine hydrochloride | CPH | 14984-68-0 | 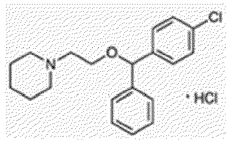 |
| Orphenadrine hydrochloride | OH | 341-69-5 | 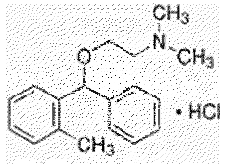 |
| Carbinoxamine maleate | CM | 3505-38-2 | 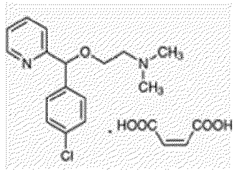 |

The purpose of comparative example 3 and examples 2 to 8 are to demonstrate the curing time of several adhesive compositions when presented as a kit comprising a cyanoacrylate adhesive component and the initiator component as deposited in or on a solid substrate (pellet). The porous pellet is as described above, i.e. a sintered porous plastic filter, made from high density polyethylene (HDPE) (for example a sintered porous HDPE filter obtainable from Porex Technologies Corp).

### Comparative Example 3: BAC control

This example demonstrates that known acyclic quaternary ammonium salts, which are also known to cause allergic contact dermatitis (ACD), especially BAC, can operate as reactive initiators for use in wound closure adhesives compositions. According to GHS statement from NIH National Library of Medicine, BAC is harmful in contact with skin (H312), BAC can cause serve skin burns and eye damage (H314), BAC is very toxic to aquatic life (H400), BAC is a dermatotoxin for skin burns, and BAC is a skin sensitizer which causes allergic reaction in the skin.

The BAC was dissolved in IPA to form a stock solution (4.43%) and 20 µL of this stock solution was added to a porous pellet to allow the porous pellet to be impregnated with BAC. The IPA was then allowed to evaporate for at least 24 hours at room temperature, resulting in a pellet containing 0.70 mg BAC (2.0*10⁻³ mmol).

The porous pellet was inserted into a connector tube as shown in Figure 1 having a threaded bore at one end and an opening at the opposite end.

The adhesive component was prepared according to the table below and filled into an aluminium tube (about 1g adhesive in the tube), followed by heat sterilization at 125°C for 65 minutes.

**Table 3, Ingredients of the adhesive component**

| Abbreviation | Chemical name | Percentage by weight |
|---|---|---|
| 2-OCA | 2-octyl cyanoacrylate | 83.89% |
| BHA | Butylated hydroxyl anisole | 0.10% |
| PHEMA | Poly (2-ethyl hexyl methacrylate) | 10.00% |
| SO2 | Sulfur dioxide | 0.01% |
| DBS | Dibutyl sebacate | 6.00% |

In use, the tube was connected to the threaded bore of the connector tube with a applicator tip (as illustrated in Figure 1). Then, the adhesive was squeezed through the connector tube to allow the adhesive to come into contact with the initiator and then exit from the applicator tip and be deposited onto the surface of a substrate such as a VWR laboratory nitrile glove (the weight of adhesive applied on the substrate was about 0.1g).

The curing time (t_{c}) was determined from the time that the adhesive exited from the applicator tip to the time that the adhesive was solidified into a tacky free polymeric film. The test was performed with n=5. The curing time was 15 seconds with standard deviation (STDEV) as 1 second.

To be noted, the curing time was too fast, and the applicator was quickly clogged due to the extreme fast polymerization.

### Example 2: DPHA

This example demonstrates that the reactivity and curing time of cyanoacrylate adhesive kit comprising DPHA as the initiator is comparable to BAC in Comparative Example 3. Additionally, tertiary amine salt initiators such as DPHA are much less toxic compared with quaternary ammonium salts such as BAC and are therefore expected to reduce the risk of allergic contact dermatitis (ACD).

The DPHA was dissolved in IPA to form a stock solution (3.71%) and 20 µL of this stock solution was added to a porous pellet to allow the porous pellet to be impregnated with DPHA. The IPA was then allowed to evaporate for at least 24 hours at room temperature, resulting in a pellet containing 0.58 mg DPHA (2.0*10⁻³ mmol).

The treated porous pellet was inserted into a connector tube as shown in Figure 1 having a threaded bore at one end and an opening at the opposite end, and in use with the same adhesive component in Comparative Example 3.

Same as the Comparative Example 3, t_{c} of the adhesive kit using DPHA as the initiator for 2-OCA based adhesive component was determined with 5 replicates. The average curing time was 42 seconds with standard deviation (STDEV) as 4 seconds.

### Example 3: CPH

This example demonstrates that the reactivity and curing time of cyanoacrylate adhesive kit comprising CPH as the initiator is comparable to BAC in Comparative Example 3. Additionally, tertiary amine salt initiators such as CPH are much less toxic compared with quaternary ammonium salts such as BAC and are therefore expected to reduce the risk of allergic contact dermatitis (ACD).

The CPH was dissolved in IPA to form a stock solution (4.66%) and 20 µL of this stock solution was added to a porous pellet to allow the porous pellet to be impregnated with CPH. The IPA was then allowed to evaporate for at least 24 hours at room temperature, resulting in a pellet containing 0.73 mg CPH (2.0*10⁻³ mmol).

The treated porous pellet was inserted into a connector tube as shown in Figure 1 having a threaded bore at one end and an opening at the opposite end, and in use with the same adhesive component in Comparative Example 3.

Same as the Comparative Example 3, t_{c} of the adhesive kit using CPH as the initiator for 2-OCA based adhesive component was determined with 5 replicates. The average curing time was 138 seconds with standard deviation (STDEV) as 11 seconds.

### Example 4: OH

This example demonstrates that the reactivity and curing time of cyanoacrylate adhesive kit comprising OH as the initiator is comparable to BAC in Comparative Example 3. Additionally, tertiary amine salt initiators such as OH are much less toxic compared with quaternary ammonium salts such as BAC and are therefore expected to reduce the risk of allergic contact dermatitis (ACD).

The OH was dissolved in IPA to form a stock solution (3.89%) and 20 µL of this stock solution was added to a porous pellet to allow the porous pellet to be impregnated with OH. The IPA was then allowed to evaporate for at least 24 hours at room temperature, resulting in a pellet containing 0.61 mg OH (2.0*10⁻³ mmol).

The treated porous pellet was inserted into a connector tube as shown in Figure 1 having a threaded bore at one end and an opening at the opposite end, and in use with the same adhesive component in Comparative Example 3.

Same as the Comparative Example 3, t_{c} of the adhesive kit using OH as the initiator for 2-OCA based adhesive component was determined with 5 replicates. The average curing time was 37 seconds with standard deviation (STDEV) as 3 seconds.

### Example 5: CM

This example demonstrates that the reactivity and curing time of cyanoacrylate adhesive kit comprising CM as the initiator is comparable to BAC in Comparative Example 3. Additionally, tertiary amine salt initiators such as CM are much less toxic compared with quaternary ammonium salts such as BAC and are therefore expected to reduce the risk of allergic contact dermatitis (ACD).

The CM was dissolved in IPA to form a stock solution (5.18%) and 20 µL of this stock solution was added to a porous pellet to allow the porous pellet to be impregnated with CM. The IPA was then allowed to evaporate for at least 24 hours at room temperature, resulting in a pellet containing 0.81 mg CM (2.0*10⁻³ mmol).

The treated porous pellet was inserted into a connector tube as shown in Figure 1 having a threaded bore at one end and an opening at the opposite end, and in use with the same adhesive component in Comparative Example 3.

Same as the Comparative Example 3, t_{c} of the adhesive kit using CM as the initiator for 2-OCA based adhesive component was determined with 5 replicates. The average curing time was 46 seconds with standard deviation (STDEV) as 1 seconds.

**Table 4, summary of single initiator**

| | Initiator | Wt of initiator in pellet (mg) | Moles of initiator in pellet (mmol) | Average curing time, t_{c} (seconds) | STDEV of curing time, t_{c} (seconds) |
|---|---|---|---|---|---|
| Comparative Example 3 | BAC | 0.70 | 2.0*10⁻³ | 15 | 1 |
| Example 2 | DPHA | 0.58 | 2.0*10⁻³ | 42 | 4 |
| Example 3 | CPH | 0.73 | 2.0*10⁻³ | 138 | 11 |
| Example 4 | OH | 0.61 | 2.0*10⁻³ | 37 | 3 |
| Example 5 | CM | 0.81 | 2.0*10⁻³ | 46 | 1 |

### Example 6: Dual initiator (DPHA and CPH)

This example demonstrates that the reactivity and curing time of cyanoacrylate adhesive kit comprising DPHA and CPH as the dual initiator is comparable to BAC in Comparative Example 3. Additionally, tertiary amine salt initiators such as both DPHA and CPH are much less toxic compared with quaternary ammonium salts such as BAC and are therefore expected to reduce the risk of allergic contact dermatitis (ACD).

The DPHA and CPH were dissolved in IPA to form a stock solution (0.76% DPHA and 3.72% CPH in IPA) and 20 µL of this stock solution was added to a porous pellet to allow the porous pellet to be impregnated with DPHA and CPH. The IPA was then allowed to evaporate for at least 24 hours at room temperature, resulting in a pellet containing 0.12 mg DPHA (0.4*10⁻³ mmol) and 0.59 mg CPH (1.6*10⁻³ mmol).

The treated porous pellet was inserted into a connector tube as shown in Figure 1 having a threaded bore at one end and an opening at the opposite end, and in use with the same adhesive component in Comparative Example 3.

Same as the Comparative Example 3, t_{c} of the adhesive kit using DPHA and CPH as the dual initiator for 2-OCA based adhesive component was determined with 5 replicates. The average curing time was 83 seconds with standard deviation (STDEV) as 9 seconds.

### Example 7: Dual initiator (DPHA and CPH)

This example demonstrates that the reactivity and curing time of cyanoacrylate adhesive kit comprising DPHA and CPH as the dual initiator is comparable to BAC in Comparative Example 3. Additionally, tertiary amine salt initiators such as both DPHA and CPH are much less toxic compared with quaternary ammonium salts such as BAC and are therefore expected to reduce the risk of allergic contact dermatitis (ACD).

The DPHA and CPH were dissolved in IPA to form a stock solution (1.81% DPHA and 2.29% CPH in IPA) and 20 µL of this stock solution was added to a porous pellet to allow the porous pellet to be impregnated with DPHA and CPH. The IPA was then allowed to evaporate for at least 24 hours at room temperature, resulting in a pellet containing 0.29 mg DPHA (1.0*10⁻³ mmol) and 0.37 mg CPH (1.0*10⁻³ mmol).

The treated porous pellet was inserted into a connector tube as shown in Figure 1 having a threaded bore at one end and an opening at the opposite end, and in use with the same adhesive component in Comparative Example 3.

Same as the Comparative Example 3, t_{c} of the adhesive kit using DPHA and CPH as the dual initiator for 2-OCA based adhesive component was determined with 5 replicates. The average curing time was 68 seconds with standard deviation (STDEV) as 5 seconds.

### Example 8: Dual initiator (DPHA and CPH)

This example demonstrates that the reactivity and curing time of cyanoacrylate adhesive kit comprising DPHA and CPH as the dual initiator is comparable to BAC in Comparative Example 3. Additionally, tertiary amine salt initiators such as both DPHA and CPH are much less toxic compared with quaternary ammonium salts such as BAC and are therefore expected to reduce the risk of allergic contact dermatitis (ACD).

The DPHA and CPH were dissolved in IPA to form a stock solution (2.96% DPHA and 0.95% CPH in IPA) and 20 µL of this stock solution was added to a porous pellet to allow the porous pellet to be impregnated with DPHA and CPH. The IPA was then allowed to evaporate for at least 24 hours at room temperature, resulting in a pellet containing 0.47 mg DPHA (1.6*10⁻³ mmol) and 0.15 mg CPH (0.4*10⁻³ mmol).

The treated porous pellet was inserted into a connector tube as shown in Figure 1 having a threaded bore at one end and an opening at the opposite end, and in use with the same adhesive component in Comparative Example 3.

Same as the Comparative Example 3, t_{c} of the adhesive kit using DPHA and CPH as the dual initiator for 2-OCA based adhesive component was determined with 5 replicates. The average curing time was 52 seconds with standard deviation (STDEV) as 8 seconds.

**Table 5, summary of dual initiator**

| | Weight of DPHA (mg) | Moles of DPHA (mmol) | Weight of CPH (mg) | Moles of CPH (mmol) | Tc, average (seconds) | Tc, STDEV (seconds) |
|---|---|---|---|---|---|---|
| Example 6 | 0.12 | 0.4*10⁻³ | 0.59 | 1.6*10⁻³ | 83 | 9 |
| Example 7 | 0.29 | 1.0*10-3 | 0.37 | 1.0*10⁻³ | 68 | 5 |
| Example 8 | 0.47 | 1.6*10⁻³ | 0.15 | 0.4*10⁻³ | 52 | 8 |

## Claims

1. A wound closure adhesive kit comprising:
a. an adhesive component comprising a cyanoacrylate; and
b. an initiator component comprising a tertiary amine salt of formula (II): 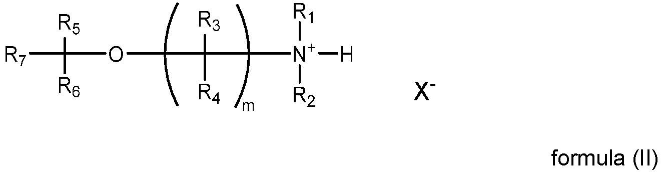 wherein:
R₁ and R₂ are each independently selected from the group consisting of: C₁₋₂₀-alkyl, aryl and heteroaryl; or
R₁ and R₂, together with the nitrogen atom to which they are attached, form a heterocycloalkyl ring having from 5 to 8 atoms;
R₃ and R₄ are both H;
R₅ and R₆ are each independently selected from the group consisting of: aryl and heteroaryl;
m is 1 - 6;
wherein any of the alkyl, heterocycloalkyl, aryl or heteroaryl groups of R₁ to R₆, can be optionally substituted with from 1 to 3 independently selected groups selected from: halo, C₁₋₄-alkyl and -(CH₂)_{q}-phenyl; wherein q is 0 - 2;
R₇ is selected from the group consisting of: H and C₁₋₂₀-alkyl; and
X⁻ is a counterion.

2. The kit according to claim 1, wherein R₁ and R₂ are each independently selected from a C₁₋₃-alkyl, optionally wherein R₁ and R₂ are each a methyl.

3. The kit according to claim 1, wherein R₁ and R₂ taken together with the nitrogen atom to which they are attached form a heterocyclic ring having 6 atoms, optionally a piperidine ring.

4. The kit according to any preceding claim, wherein m is 1 to 3; optionally wherein m is 2.

5. The kit according to any preceding claim, wherein R₅ and R₆ are selected from the group consisting of: phenyl and pyridinyl.

6. The kit according to any preceding claim, wherein R₇ is selected from the group consisting of: H and C₁₋₃-alkyl.

7. The kit according to any preceding claim , wherein X⁻ is selected from halide, maleate, acetate or succinate; optionally wherein X⁻ is halide; further optionally wherein X⁻ is chloride.

8. The kit according to claim 1, wherein the tertiary amine salt is selected from the group consisting of: diphenylhydramine hydrochloride, orphenadrine hydrochloride, carbinoxamine maleate, cloperastine hydrochloride and doxylamine succinate.

9. The kit according to any preceding claim, wherein the cyanoacrylate component has the formula (IV): 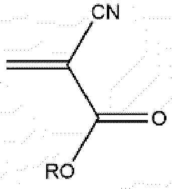 wherein R is C₁-C₂₀ alkyl, C₂-C₂₀ alkenyl or C₂-C₂₀ alkynyl, wherein R is optionally substituted with one or more moieties selected from the group consisting of: -O-C₁-C₁₀ alkyl, -C(O)O-C₁-C₁₀ alkyl, aryl and heteroaryl, wherein any of the alkyl, alkenyl, alkynyl, aryl or heteroaryl groups can be optionally substituted with from 1 to 6 independently selected halo groups.

10. The kit according to claim 9, wherein R is C₁-C₁₅ alkyl.

11. The kit according to claim 9 or claim 10, wherein the cyanoacrylate component is selected from 2-octylcyanoacrylate, n-2-butyl-cyanoacrylate or 2-ethylcyanoacrylate; optionally wherein the cyanoacrylate component is 2-octylcyanoacrylate.

12. The kit according to any preceding claim, wherein the kit comprises a mixture of two or more tertiary amine salts as defined in any one of claims 1 to 8.

13. The kit according to any preceding claim, wherein the kit further comprises an applicator in which the at least one tertiary amine salt is contained; and/or wherein the initiator component comprises a porous solid substrate, and wherein the tertiary amine salt is deposited onto and/or into the solid substrate.

14. A wound closure adhesive composition comprising:
a. a cyanoacrylate; and
b. a tertiary amine salt;
wherein the cyanoacrylate and tertiary amine salt are as defined in any of claims 1 to 12.

15. The use of a tertiary amine salt as an initiator in a cyanoacrylate wound closure adhesive wherein the cyanoacrylate adhesive and tertiary amine salt are as defined in any of claims 1 to 12.

16. An adhesive composition comprising a cyanoacrylate adhesive and a tertiary amine salt for use in a method of applying a cyanoacrylate wound closure adhesive comprising:
a. contacting the cyanoacrylate adhesive with a tertiary amine salt;
b. depositing the cyanoacrylate wound closure adhesive on a surface;
wherein the cyanoacrylate adhesive and tertiary amine salt are as defined in any of claims 1 to 12.

17. A wound closure adhesive product comprising:
a. a sealed first vessel containing a cyanoacrylate adhesive component; and
b. a second vessel containing an initiator component;
wherein the cyanoacrylate adhesive component and initiator component are as defined in any of claims 1 to 13.

## Patentansprüche

1. Wundverschlussklebstoffkit, umfassend:
a. eine Klebstoffkomponente, die ein Cyanoacrylat umfasst; und
b. eine Initiatorkomponente, die ein tertiäres Aminsalz der Formel (II) umfasst: 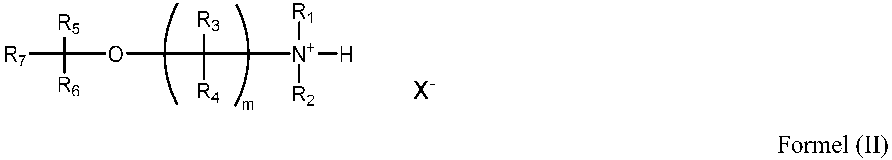 wobei:
R₁ und R₂ jeweils unabhängig ausgewählt sind aus der Gruppe bestehend aus: C₁₋₂₀-Alkyl, Aryl und Heteroaryl; oder
R₁ und R₂ zusammen mit dem Stickstoffatom, an das sie gebunden sind, einen Heterocycloalkylring mit 5 bis 8 Atomen ausbilden;
R₃ und R₄ beide H sind;
R₅ und R₆ jeweils unabhängig ausgewählt sind aus der Gruppe bestehend aus: Aryl und Heteroaryl;
m 1 - 6 ist;
wobei beliebige der Alkyl-, Heterocycloalkyl-, Aryl- oder Heteroarylgruppen von R₁ bis R₆ optional substituiert sein können mit 1 bis 3 unabhängig ausgewählten Gruppen ausgewählt aus:
Halogen, C₁₋₄-Alkyl und -(CH₂)_{q}-Phenyl; wobei q 0 - 2 ist;
R₇ aus der Gruppe ausgewählt ist, die aus Folgendem besteht: H und C₁₋₂₀-Alkyl; und
X⁻ ein Gegenion ist.

2. Kit nach Anspruch 1, wobei R₁ und R₂ jeweils unabhängig ausgewählt sind aus einem C₁₋₃-Alkyl, optional wobei R₁ und R₂ jeweils ein Methyl sind.

3. Kit nach Anspruch 1, wobei R₁ und R₂ zusammen mit dem Stickstoffatom, an das sie gebunden sind, einen heterocyclischen Ring mit 6 Atomen, optional einen Piperidinring, ausbilden.

4. Kit nach einem vorhergehenden Anspruch, wobei m 1 bis 3 ist; optional wobei m 2 ist.

5. Kit nach einem vorhergehenden Anspruch, wobei R₅ und R₆ ausgewählt sind aus der Gruppe bestehend aus: Phenyl und Pyridinyl.

6. Kit nach einem vorhergehenden Anspruch, wobei R₇ ausgewählt ist aus der Gruppe bestehend aus: H und C₁₋₃-Alkyl.

7. Kit nach einem vorhergehenden Anspruch , wobei X⁻ ausgewählt ist aus Halogenid, Maleat, Acetat oder Succinat; optional
wobei X⁻ Halogenid ist; ferner optional wobei X⁻ Chlorid ist.

8. Kit nach Anspruch 1, wobei das tertiäre Aminsalz aus der Gruppe ausgewählt ist, die aus Folgendem besteht: Diphenylhydraminhydrochlorid, Orphenadrinhydrochlorid, Carbinoxaminmaleat, Cloperastinhydrochlorid und Doxylaminsuccinat.

9. Kit nach einem vorhergehenden Anspruch, wobei die Cyanoacrylatkomponente die Formel (IV) aufweist: 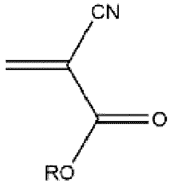 wobei R C₁-C₂₀-Alkyl, C₂-C₂₀-Alkenyl oder C₂-C₂₀-Alkinyl ist, wobei R optional mit einer oder mehreren Einheiten substituiert ist, ausgewählt aus der Gruppe bestehend aus: -O-C₁-C₁₀-Alkyl, -C(O)O-C₁-C₁₀-Alkyl, Aryl und Heteroaryl, wobei beliebige der Alkyl-, Alkenyl-, Alkinyl-, Aryl- oder Heteroarylgruppen optional mit 1 bis 6 unabhängig ausgewählten Halogengruppen substituiert sein können.

10. Kit nach Anspruch 9, wobei R C₁-C₁₅-Alkyl ist.

11. Kit nach Anspruch 9 oder Anspruch 10, wobei die Cyanoacrylatkomponente ausgewählt ist aus 2-Octylcyanoacrylat, n-2-Butylcyanoacrylat oder 2-Ethylcyanoacrylat;
optional wobei die Cyanoacrylatkomponente 2-Octylcyanoacrylat ist.

12. Kit nach einem vorhergehenden Anspruch, wobei das Kit eine Mischung von zwei oder mehr tertiären Aminsalzen, wie in einem der Ansprüche 1 bis 8 definiert, umfasst.

13. Kit nach einem vorhergehenden Anspruch, wobei das Kit ferner einen Applikator umfasst, in dem das mindestens eine tertiäre Aminsalz enthalten ist; und/oder
wobei die Initiatorkomponente ein poröses festes Substrat umfasst und wobei das tertiäre Aminsalz auf und/oder in dem festen Substrat abgeschieden ist.

14. Wundverschlussklebstoffzusammensetzung, umfassend:
a. ein Cyanoacrylat; und
b. ein tertiäres Aminsalz;
wobei das Cyanoacrylat und das tertiäre Aminsalz wie in einem der Ansprüche 1 bis 12 definiert sind.

15. Verwendung eines tertiären Aminsalzes als einen Initiator in einem Cyanoacrylat-Wundverschlussklebstoff, wobei der Cyanoacrylat-Klebstoff und das tertiäre Aminsalz wie in einem der Ansprüche 1 bis 12 definiert sind.

16. Klebstoffzusammensetzung, umfassend einen Cyanoacrylat-Klebstoff und ein tertiäres Aminsalz zur Verwendung in einem Verfahren eines Auftragens eines Cyanoacrylat-Wundverschlussklebstoffs, umfassend:
a. Inkontaktbringen des Cyanoacrylat-Klebstoffs mit einem tertiären Aminsalz;
b. Abscheiden des Cyanoacrylat-Wundverschlussklebstoffs auf einer Oberfläche;
wobei der Cyanoacrylat-Klebstoff und das tertiäre Aminsalz wie in einem der Ansprüche 1 bis 12 definiert sind.

17. Wundverschlussklebstoffprodukt, umfassend:
a. einen versiegelten ersten Behälter, der eine Cyanoacrylat-Klebstoffkomponente enthält; und
b. einen zweiten Behälter, der eine Initiatorkomponente enthält;
wobei die Cyanoacrylat-Klebstoffkomponente und die Initiatorkomponente wie in einem der Ansprüche 1 bis 13 definiert sind.

## Revendications

1. Kit d'adhésif de fermeture de plaies comprenant :
a. un composant adhésif comprenant un cyanoacrylate ; et
b. un composant initiateur comprenant un sel d'amine tertiaire de formule (II) : 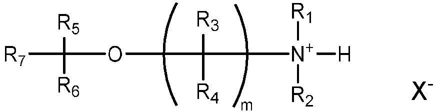 formule (II) où :
R₁ et R₂ sont chacun indépendamment choisis dans le groupe constitué par : un alkyle en C₁₋₂₀, un aryle et un hétéroaryle ; ou
R₁ et R₂, conjointement avec l'atome d'azote auquel ils sont liés, forment un cycle hétérocycloalkyle comportant 5 à 8 atomes ;
R₃ et R₄ sont tous deux H ;
R₅ et R₆ sont chacun indépendamment choisis dans le groupe constitué par : un aryle et un hétéroaryle ;
m vaut de 1 à 6 ;
où l'un quelconque des groupes alkyle, hétérocycloalkyle, aryle ou hétéroaryle de R₁ à R₆, peut être éventuellement substitué par 1 à 3 groupes indépendamment choisis parmi : un halogéno, un alkyle en C₁₋₄ et -(CH₂)_{q}-phényle ; où q vaut de 0 à 2 ;
R₇ est choisi dans le groupe constitué par : H et un alkyle en C₁₋₂₀ ; et
X⁻ est un contre-ion.

2. Kit selon la revendication 1, dans lequel R₁ et R₂ sont chacun indépendamment choisis parmi un alkyle en C₁₋₃, éventuellement dans lequel R₁ et R₂ sont chacun un méthyle.

3. Kit selon la revendication 1, dans lequel R₁ et R₂ pris conjointement avec l'atome d'azote auquel ils sont liés forment un cycle hétérocyclique comportant 6 atomes, éventuellement un cycle pipéridine.

4. Kit selon une quelconque revendication précédente, dans lequel m vaut de 1 à 3 ; dans lequel, éventuellement, m vaut 2.

5. Kit selon une quelconque revendication précédente, dans lequel R₅ et R₆ sont choisis dans le groupe constitué par : un phényle et un pyridinyle.

6. Kit selon une quelconque revendication précédente, dans lequel R₇ est choisi dans le groupe constitué par : H et un alkyle en C₁₋₃.

7. Kit selon une quelconque revendication précédente , dans lequel ^{X-} est choisi parmi un halogénure, un maléate, un acétate ou un succinate ; éventuellement
dans lequel X⁻ est un halogénure ; en outre, éventuellement, dans lequel X⁻ est un chlorure.

8. Kit selon la revendication 1, dans lequel le sel d'amine tertiaire est choisi dans le groupe constitué par : le chlorhydrate de diphénylhydramine, le chlorhydrate d'orphénadrine, le maléate de carbinoxamine, le chlorhydrate de clopérastine et le succinate de doxylamine.

9. Kit selon une quelconque revendication précédente, dans lequel le composant cyanoacrylate présente la formule (IV) : 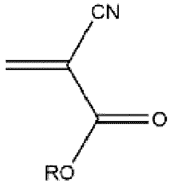 où R est un alkyle en C₁-C₂₀, un alcényle en C₂-C₂₀ ou un alcynyle en C₂-C₂₀, où R est éventuellement substitué par un ou plusieurs fragments choisis dans le groupe constitué par : -O-alkyle en C₁-C₁₀, -C(O)O-alkyle en C₁-C₁₀, un aryle et un hétéroaryle, où l'un quelconque des groupes alkyle, alcényle, alcynyle, aryle ou hétéroaryle peut être éventuellement substitué par 1 à 6 groupes halogéno indépendamment choisis.

10. Kit selon la revendication 9, dans lequel R est un alkyle en C₁-C₁₅.

11. Kit selon la revendication 9 ou la revendication 10, dans lequel le composant cyanoacrylate est choisi parmi le 2-octylcyanoacrylate, le n-2-butylcyanoacrylate ou le 2-éthylcyanoacrylate ; éventuellement dans lequel le composant cyanoacrylate est le 2-octylcyanoacrylate.

12. Kit selon une quelconque revendication précédente, dans lequel le kit comprend un mélange de deux sels d'amines tertiaires ou plus tels que définis dans l'une quelconque des revendications 1 à 8.

13. Kit selon une quelconque revendication précédente, dans lequel le kit comprend en outre un applicateur dans lequel l'au moins un sel d'amine tertiaire est contenu ; et/ou dans lequel le composant initiateur comprend un substrat solide poreux, et dans lequel le sel d'amine tertiaire est déposé sur et/ou dans le substrat solide.

14. Composition adhésive de fermeture de plaies comprenant :
a. un cyanoacrylate ; et
b. un sel d'amine tertiaire ;
dans laquelle le cyanoacrylate et le sel d'amine tertiaire sont tels que définis dans l'une quelconque des revendications 1 à 12.

15. Utilisation d'un sel d'amine tertiaire en tant qu'initiateur dans un adhésif cyanoacrylate de fermeture de plaies, dans laquelle l'adhésif cyanoacrylate et le sel d'amine tertiaire sont tels que définis dans l'une quelconque des revendications 1 à 12.

16. Composition adhésive comprenant un adhésif cyanoacrylate et un sel d'amine tertiaire destinée à être utilisée dans un procédé d'application d'un adhésif cyanoacrylate de fermeture de plaies comprenant :
a. la mise en contact de l'adhésif cyanoacrylate avec un sel d'amine tertiaire ;
b. le dépôt de l'adhésif cyanoacrylate de fermeture de plaies sur une surface ;
dans laquelle l'adhésif cyanoacrylate et le sel d'amine tertiaire sont tels que définis dans l'une quelconque des revendications 1 à 12.

17. Produit adhésif de fermeture de plaies comprenant :
a. un premier récipient scellé contenant un composant adhésif cyanoacrylate ; et
b. un second récipient contenant un composant initiateur ;
dans lequel le composant adhésif cyanoacrylate et le composant initiateur sont tels que définis dans l'une quelconque des revendications 1 à 13.
